# EUROPÄISCHE PATENTSCHRIFT

(11) **EP 1 843 807 B1**
(45) Veröffentlichungstag und Bekanntmachung des Hinweises auf die Patenterteilung: **17.08.2016**
(21) Anmeldenummer: 05809810.4
(22) Anmeldetag: 07.12.2005
(51) Int. Cl.: A61M 1/06

(54) **BRUSTPUMPENSET**
BREAST PUMP SET
ENSEMBLE TIRE-LAIT

(30) Priorität: 28.01.2005 CH 140052005
(43) Veröffentlichungstag der Anmeldung: 17.10.2007
(73) Patentinhaber: Medela Holding AG, 6340 Baar (CH)
(72) Erfinder: THOMMEN, Daniel, CH-6312 Steinhausen (CH)
(74) Vertreter: Clerc, Natalia
(86) Internationale Anmeldenummer: PCT/CH2005/000730
(87) Internationale Veröffentlichungsnummer: WO 2006/079229

(56) Entgegenhaltungen:
- DE-U1- 20 319 642
- US-A- 5 542 921
- US-A- 6 004 288
- US-A1- 2003 040 734
- US-B1- 6 673 036

## Beschreibung

### Technisches Gebiet

Die Erfindung betrifft eine Brusthaube und ein Brustpumpenset gemäss Oberbegriff des Patentanspruchs 1 bzw. 8.

### Stand der Technik

Brustpumpen zum Abpumpen von menschlicher Muttermilch sind hinlänglich bekannt. Grundsätzlich gibt es zwei verschiedene Typen: die ersten werden manuell bedient, d.h. der für das Abpumpen notwendige Unterdruck wird durch manuelle Betätigung der Saugpumpe erzeugt. Bei den zweiten Typen ist die Saugpumpe elektrisch betrieben, wobei die Saugpumpe ans Stromversorgungsnetz angeschlossen sein kann und/oder über eine Batterie bzw. einen anderen Energiespeicher betrieben werden kann.

Damit die Funktion der Brustpumpe optimal an die Bedürfnisse der Mutter angepasst werden kann, bieten einige der Pumpen der Mutter die Möglichkeit, den Unterdruck zu regulieren. So offenbaren US 2004/0024351 und US-A-4'813'932 manuell betätigte Brustpumpen, welche einstellbare Ventile aufweisen. Die Saugleistung der Pumpe selber wird bei manuell betriebenen Pumpen zudem dadurch geregelt, dass die Mutter entsprechend ihren Bedürfnissen die Pumpe stärker oder schwächer betätigt.

Auch bei elektrisch betriebenen Pumpen ist es üblich, an der Brusthaube oder an der Verbindungsleitung zur Saugpumpe Entlüftungsmöglichkeiten anzubringen. Dies ist beispielsweise bei US 6'706'012 und US 6'042'560 der Fall.

US 6'110'140 schlägt eine manuell oder elektrisch betriebene Brustpumpe vor, die im Bereich der Brusthaube einen Vakuumregulator aufweist, welcher den durch die Saugpumpe erzeugten Unterdruck reguliert. Dieser Regulator lässt sich während des Gebrauchs der Brustpumpe betätigen, so dass der in der Brusthaube herrschende Unterdruck angepasst werden kann.

Zusätzlich zu den oben beschriebenen Lösungen, welche alle den von der Saugpumpe erzeugten Unterdruck im Bereich der Brusthaube ändern können, verfügen einige elektrisch betriebene Brustpumpen zudem über die Möglichkeit, den Unterdruck bzw. den Saugrhythmus am Pumpengerät selber zu ändern. Dies erfolgt über entsprechende Betätigungsschalter oder -knöpfe, welche an der Saugpumpe angeordnet sind. Um diese Knöpfe betätigen zu können, muss jedoch die Mutter eine Hand frei haben. Dies ist jedoch insbesondere dann nicht möglich, wenn die Mutter beide Brüste gleichzeitig abpumpen möchte.

### Darstellung der Erfindung

Es ist deshalb eine Aufgabe der Erfindung, der Mutter eine möglichst einfache Bedienung der Saugpumpe zu ermöglichen.

Diese Aufgabe löst eine Brusthaube sowie ein Brustpumpenset mit den Merkmalen des Patentanspruchs 1 bzw. 8.

Das erfindungsgemässe Brustpumpenset zum Abpumpen menschlicher Muttermilch weist eine Brusthaube zur Anlage an eine Mutterbrust, einen Milchauffangbehälter und eine elektrisch betriebene Saugpumpe auf, wobei die Brusthaube über ein Bedienungsmittel zur Bedienung der Saugpumpe verfügt.

Dank dem erfindungsgemässen Brustpumpenset kann somit die Mutter die Funktion der Saugpumpe beeinflussen, ohne dass diese für sie erreichbar sein muss. Die Mutter kann dieselbe Hand verwenden, mit welcher sie die Brusthaube an ihre Brust hält. Sie kann somit beide Brüste gleichzeitig abpumpen oder die freie Hand für etwas anderes benützen.

Vorzugsweise ist das Bedienungsmittel nahe beim Brusthaubentrichter angeordnet.

In bevorzugten Ausführungsformen ist das Bedienungsmittel in Gebrauchslage der Brusthaube oben oder seitlich angeordnet, damit es gut zugänglich ist. Je nach Form und Ausgestaltung der Brusthaube sind jedoch auch andere Stellen möglich, um das Bedienungsmittel anzuordnen, wobei gut zugängliche und der natürlichen Haltung der Mutter beim Abpumpen der Milch entsprechende Lagen zu bevorzugen sind. Das Bedienungsmittel lässt sich zudem auch auf einem aufklappbaren, aufsteckbaren oder ausziehbaren Teil der Brusthaube anordnen, so dass das Bedienungsmittel nur freigelegt ist, wenn die Mutter davon Gebrauch machen will.

Je nach Ausführungsform lässt sich die Saugpumpe mittels des Bedienungsmittels einund ausschalten. Zusätzlich oder alternativ kann auch ihre Pumpleistung entsprechend den Bedürfnissen der Mutter geändert werden.

Wird eine Saugpumpe verwendet, welche gleichzeitig zwei Brusthauben bedienen kann, so ist je nach Ausführungsform der Saugpumpe nur eine der Brusthauben mit dem Bedienungsmittel versehen und die Saugpumpe bedient beide Brusthauben nach Massgabe von denselben Bediensignalen. Ist die Saugpumpe in der Lage, die zwei Brusthauben mit unterschiedlichen Saugrhythmen oder Pumpleistungen zu beaufschlagen, so können beide Brusthauben mit einem Bedienungsmittel versehen sein, um die Saugpumpe individuell einzustellen.

Die Übermittlung der Signale vom Bedienungsmittel zur Saugpumpe kann mechanisch, drahtlos oder über entsprechende Kabelverbindungen erfolgen. Bei drahtloser Übermittlung lässt sich beispielsweise eine Funkantenne, eine Infrarotschnittstelle, Bluetooth-Signalübermittlung oder eine andere der bekannten drahtlosen Übermittlungsarten verwenden. Die Kabelverbindung erfolgt vorzugsweise über ein elektrisches Signal in bekannter Art und Weise.

Es ist auch möglich, anstelle eines Bedienungsmittels ein Spracherkennungsmittel zu verwenden, welches anschliessend ein Steuersignal an die Saugpumpe übermittelt. Dieses Spracherkennungsmittel kann im Bereich der Brusthaube, direkt an der Saugpumpe oder an einer mit der Saugpumpe verbundenen zusätzlichen externen Einheit angeordnet sein.

Vorzugsweise weist die Brusthaube einen einstückig ausgebildeten Grundkörper auf, in welchem das Bedienungsmittel und allfällige Verbindungsleitungen zur Signalübermittlung angeordnet sind. Um die Kosten für derartige Brusthauben möglichst gering zu halten, lässt sich der Grundkörper jedoch auch mehrteilig ausbilden, wobei beispielsweise ein Brusthaubentrichter als Einwegprodukt ausgebildet ist und zumindest der Teil mit dem Bedienungsmittel als Mehrwegprodukt wiederverwendet werden kann.

Weitere vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Es zeigen:
- Figur 1: eine Darstellung eines Brustpumpensets gemäss einer ersten bevorzugten Ausführungsform der Erfindung;
- Figur 2: eine Darstellung eines Brustpumpensets gemäss einer zweiten bevorzugten Ausführungsform der Erfindung;
- Figur 3: eine Darstellung eines Brustpumpensets gemäss einer dritten bevorzugten Ausführungsform der Erfindung;
- Figur 4: eine Darstellung eines Brustpumpensets gemäss einer vierten bevorzugten Ausführungform der Erfindung;
- Figur 5: eine Darstellung einer Brusthaube gemäss einer Ausführungsform der Erfindung und
- Figur 6: eine Darstellung einer Brusthaube gemäss einer weiteren Ausführungsform.

### Wege zur Ausführung der Erfindung

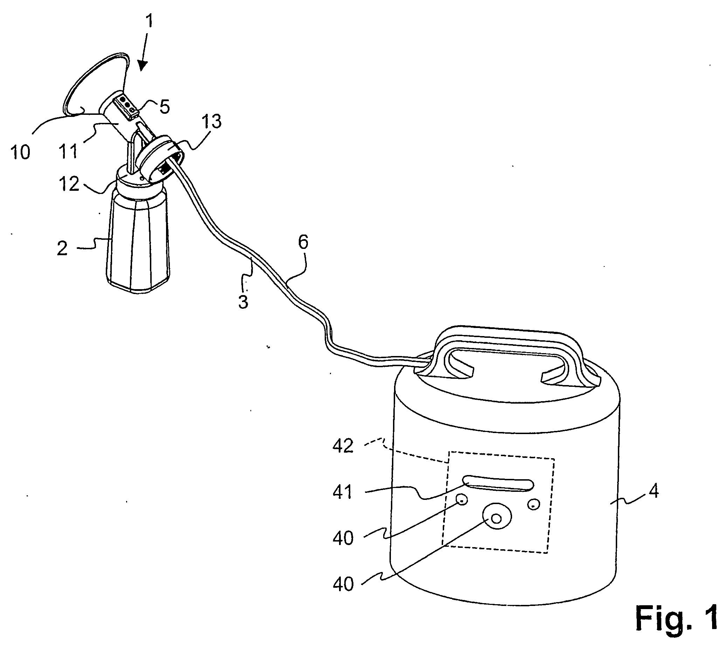

In Figur 1 ist eine erste bevorzugte Ausführungsform des erfindungsgemässen Brustpumpensets dargestellt. Es weist im wesentlichen mindestens eine Brusthaube 1, einen an die Brusthaube 1 befestigbaren Milchauffangbehälter 2 und eine elektrisch betriebene Saugpumpe 4 auf, welche über eine Saugleitung 3 mit der Brusthaube 1 verbunden ist.

Die Brusthaube 1 weist einen Brusthaubentrichter 10 auf, welcher bei Gebrauch an die Brust der Mutter angelegt wird. Der Trichter 10 geht in einen Brusthaubenhals 11 über, an welchem ein erstes und ein zweites Brusthaubenkopplungsteil 12, 13 angrenzen. Das erste Brusthaubenkopplungsteil 12 weist ein Innengewinde auf, welches sich auf ein Aussengewinde des Milchsammelbehälters 2, hier eine Babyflasche, aufschrauben lässt. Das zweite Brusthaubenkopplungsteil 13 weist ein in der Figur nicht sichtbares Anschlussmittel auf, in welches die Saugleitung 3, hier ein Schlauch, einsteckbar ist. Das andere Ende des Schlauchs ist in der Saugpumpe 4 einsteckbar. Über diesen Schlauch wird das in der Saugpumpe erzeugte Vakuum bzw. der dort erzeugte Unterdruck an die Brusthaube angelegt, so dass die Milch aus der Mutterbrust abgepumpt und im Milchsammelbehälter 2 gesammelt werden kann.

In Figur 1 ist die übliche Gebrauchslage dargestellt. Das erste Kopplungsteil 12 ist nach unten gerichtet, das zweite Kopplungsteil 13 nach hinten oder schräg nach unten, auf jeden Fall von der Mutterbrust weg.

Die Saugpumpe 4 kann eine der bekannten motorisch angetriebenen Saugpumpen sein. Vorzugsweise verfügt sie über Betätigungs- und Reguliertasten bzw. -knöpfe 40 und eine Anzeige 41. Im hier dargestellten Ausführungsbeispiel ist sie als eigenständiges, portables Gerät ausgebildet.

Erfindungsgemäss weist nun die Brusthaube 1 ein Bedienungsmittel, hier in Form eines Bedienfelds oder -paneels 5, auf, über welches die Saugpumpe 4 bedient werden kann. Vorzugsweise erfolgt diese Bedienung unabhängig davon, wie die direkt an der Saugpumpe 4 angeordneten Betätigungs- und Reguliertasten bzw. -knöpfe 40 eingestellt sind.

Die Bedienung kann das Ein- und Ausschalten der Saugpumpe 4, jedoch auch die Einstellung des Saugrhythmus, der Saugkurve, der Saugleistung, der Intervalle zwischen einzelnen Zyklen usw. umfassen.

Das Bedienpanel 5 ist vorzugsweise im Bereich des Brusthaubentrichters 10 angeordnet. Im hier dargestellten Beispiel ist es angrenzend an den Trichter 10 auf dem Brusthaubenhals 11 befestigt bzw. in diesem eingelassen. Vorzugsweise ist es in der hier dargestellten Gebrauchslage nach oben gerichtet, so dass es mit derselben Hand erreichbar ist, mit welcher die Mutter die Brusthaube 1 an ihre Brust hält. Das Bedienpanel 5 kann jedoch beispielsweise auch seitlich oder unten am Hals 11 angeordnet sein.

Die Befestigung des Bedienpanels 5 auf oder in der Brusthaube 1 erfolgt mittels bekannten Mitteln. Der Grundkörper der Brusthaube 1 selber ist üblicherweise aus Kunststoff gefertigt und ein- oder mehrstückig ausgebildet. Das Bedienpanel 5 kann beispielsweise in diesen Grundkörper eingegossen sein oder auf ihm aufgeklebt sein. Im zweiten Fall kann der Grundkörper eine entsprechende Ausnehmung zur steckbaren Aufnahme des Bedienpanels 5 aufweisen, damit dieses nicht allzu sehr vorsteht und somit geschützt ist. Allfällige Kabel zur Signalübertragung lassen sich auf dieselbe Weise mit der Brusthaube 1 verbinden bzw. in diese einlassen.

Das Bedienpanel 5 kann über Schalter, Drucktasten, Sensortasten, Drehknöpfe, Schieber oder andere bekannte Mittel verfügen, damit die Mutter die gewünschten Steuersignale an die Saugpumpe 4 bzw. an die darin angeordnete Steuerung 42 übermitteln kann.

Im hier dargestellten Ausführungsbeispiel erfolgt die Übermittlung der Signale von der Brusthaube 1 zur Saugpumpe 4 über eine Signalleitung 6. Diese Leitung kann getrennt von der Saugleitung 3 verlaufen und auch über andere Kontaktstellen mit der Brusthaube 1 bzw. der Saugpumpe 4 verbunden sein. Vorzugsweise wird jedoch ein Verbindungsschlauch eingesetzt, welcher sowohl Saugleitung 3 wie auch Signalleitung 6 beinhaltet. Je nach Ausführungsform sind mehr als eine Saugleitung 3 bzw. Signalleitung 6 vorhanden. Die mindestens zwei Leitungen 3, 6 sind vorzugsweise über ihre gesamte Länge miteinander verbunden. Beispielsweise sind sie koextrudiert oder werden nach ihrer getrennten Herstellung gemeinsam ummantelt. Sie weisen an beiden Enden Steckverbindungen auf, welche in entsprechende Steckeraufnahmen der Brusthaube 1 und der Saugpumpe 4 einsteckbar sind.

Werden zwei Brusthauben 1 an dieselbe Saugpumpe 4 angeschlossen, sind selbstverständlich zwei Verbindungsschläuche mit Saugleitungen 3 vorhanden, wobei beide Schläuche oder nur einer davon zusätzlich noch eine Signalleitung 6 aufweisen kann.

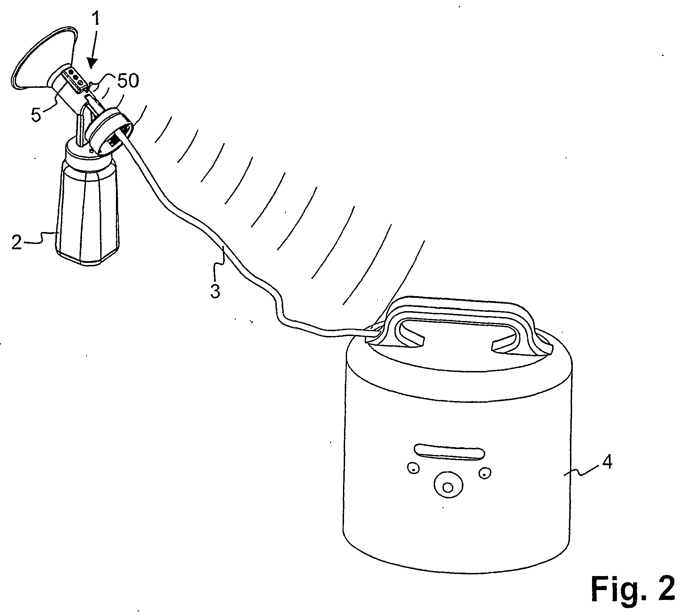

In Figur 2 ist eine alternative Ausführungsform dargestellt. Gleiche Teile sind mit denselben Bezugszeichen versehen. Hier erfolgt nun die Signalübermittlung von der Brusthaube 1 zur Saugpumpe 4 nicht über eine Leitung, sondern drahtlos, vorzugsweise per Funk. Stellvertretend für alle Möglichkeiten der drahtlosen Übermittlung ist eine Antenne 50 gezeichnet, wobei darauf hingewiesen wird, dass die heute zur Verfügung stehenden Antennen im allgemeinen flächig in die Brusthaube 1 integriert werden können.

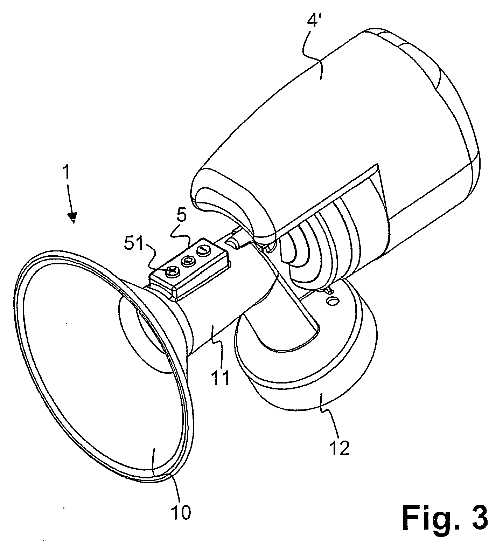

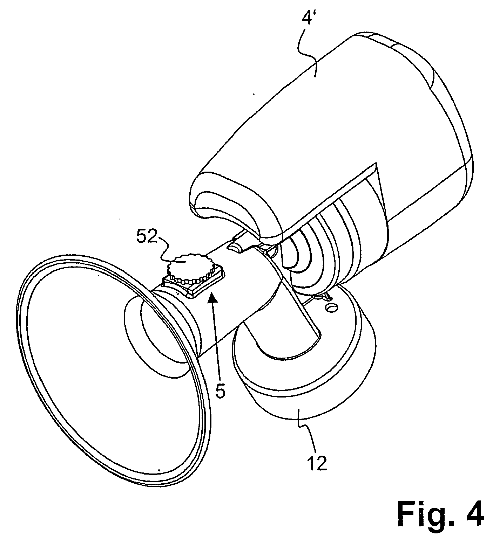

In den Ausführungsformen gemäss den Figuren 3 und 4 ist der Milchauffangbehälter 2 nicht dargestellt. Er wird jedoch auf bekannte Art und Weise an das erste Kopplungsteil 12 angeschraubt. Im Gegensatz zu den obigen Ausführungsformen ist hier nun die Saugpumpe eine direkt am zweiten Kopplungsteil 13 der Brusthaube 1 befestigte Saugpumpeneinheit 4'. Derartige Saugpumpen verfügen üblicherweise über eine Batterie oder einen anderen Energiespeicher, welcher ebenfalls in dieser Einheit 4' angeordnet ist. Die Befestigung der Saugpumpeneinheit 4' an die Brusthaube 1 erfolgt auf bekannte Art und Weise, beispielsweise über eine Schraubverbindung, Klemmmittel oder andere form- bzw. kraftschlüssige Befestigungsmittel.

Um eine gute Erreichbarkeit mit den Fingern zu erhalten, ist das Bedienpanel 5 der Pumpe 4' nicht auf der Einheit selber, sondern nach wie vor auf der Brusthaube 1, vorzugsweise im Bereich des Trichters 10, angeordnet. Auch hier hat sich als besonders geeigneter Ort der Hals 11 der Haube 1 erwiesen.

In Figur 3 weist das Bedienpanel 5 mindestens eine, vorzugsweise mehrere Tasten 51 auf, in Figur 4 mindestens einen Drehknopf 52.

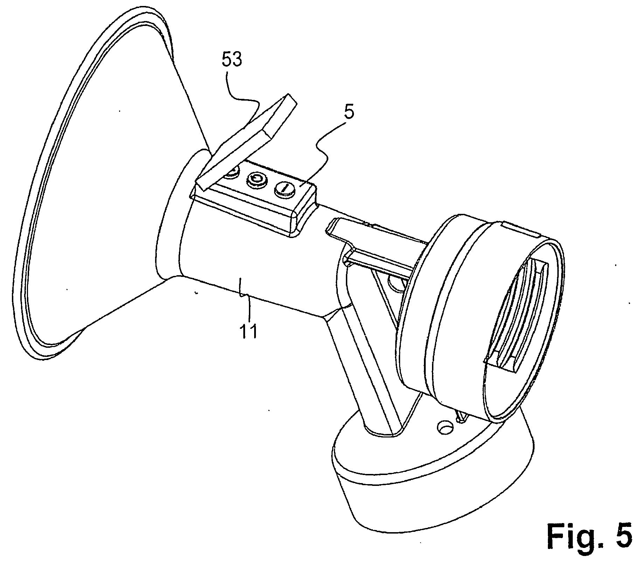

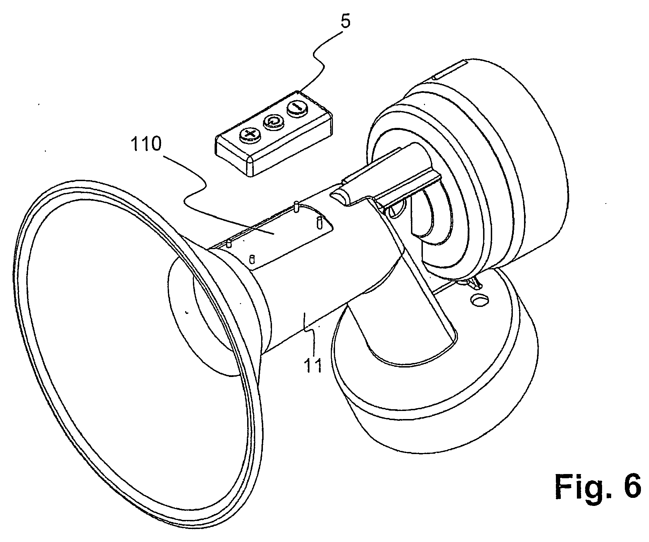

In der Ausführungsform gemäss Figur 5 ist das Bedienpaneel 5 mit einer aufklappbaren Klappe 53 geschützt. In Figur 6 ist das Bedienpaneel 5 aufsteckbar, wobei der Brusthaubenhals 11 eine entsprechende Ausnehmung 100 mit Aufnahmestiften aufweist, welche in Befestigungslöcher des Panels 5 eingreifen..

Die Signalübermittlung zur Saugpumpeneinheit 4' kann wiederum über Signalleitungen oder drahtlos erfolgen. Falls Signalleitungen verwendet werden, wird vorzugsweise der Kontakt bzw. die Verbindung der in der Brusthaube 1 und der Saugpumpeneinheit 4' verlaufenden Leitungsabschnitte automatisch hergestellt, wenn die Pumpe 4' an die Haube 1 angekoppelt wird.

Die erfindungsgemässe Brusthaube ermöglicht somit eine einfache Bedienung der Saugpumpe, ohne dass die Mutter hierzu eine Hand frei haben muss.

### Bezugszeichenliste

- 1: Brusthaube
- 10: Brusthaubentrichter
- 11: Brusthaubenhals
- 110: Ausnehmung
- 12: Erstes Brusthaubenkopplungsteil
- 13: Zweites Brusthaubenkopplungsteil
- 2: Milchauffangbehälter
- 3: Saugleitung
- 4: Saugpumpengerät
- 4': Saugpumpeneinheit
- 40: Reguliertasten bzw. -knöpfe
- 41: Anzeige
- 42: Steuerung
- 5: Bedienpaneel
- 50: Antenne
- 51: Taste
- 52: Drehknopf
- 53: Klappe
- 6: Signalleitung

## Patentansprüche

1. Brusthaube eines Brustpumpensets zum Abpumpen von menschlicher Muttermilch, wobei die Brusthaube (1) einen Brusthaubentrichter (10) zur Anlage an eine Mutterbrust, einen ersten Kopplungsteil (12) zur Verbindung mit einem Milchsammelbehälter (2) und einem zweiten Kopplungsteil (13) zur Verbindung mit einer Saugpumpe (4, 4') sowie ein Bedienungsmittel aufweist, **dadurch gekennzeichnet, dass** das Bedienungsmittel ein Bedienungsmittel (5) zur Bedienung der Saugpumpe (4, 4') ist.

2. Brusthaube nach Anspruch 1, wobei der zweite Kopplungsteil (13) über eine Saugleitung (3) mit der Saugpumpe (4) verbindbar ist.

3. Brusthaube nach Anspruch 1, wobei der zweite Kopplungsteil (13) Mittel zur Befestigung der Saugpumpe (4') aufweist.

4. Brusthaube nach einem der Ansprüche 1 bis 3, wobei das Bedienungsmittel (5) im Bereich des Brusthaubentrichters (10) angeordnet ist.

5. Brusthaube nach Anspruch 4, wobei das Bedienungsmittel (5) auf einem an die Brusthaube (1) angrenzenden Brusthaubenhals (11) angeordnet ist.

6. Brusthaube nach einem der Ansprüche 4 oder 5, wobei im Gebrauchszustand der erste Kopplungsteil (12) nach unten gerichtet ist und das Bedienungsmittel (5) auf einem nach oben gerichteten oder nach unten gerichteten oder seitlich gerichteten Bereich der Brusthaube (1) angeordnet ist.

7. Brusthaube nach einem der Ansprüche 1 bis 6, wobei die Brusthaube (1) einen aufsteckbaren, aufklappbaren oder ausziehbaren Teil aufweist, auf welchem das Bedienungsmittel (5) angeordnet ist.

8. Brustpumpenset zum Abpumpen von menschlicher Muttermilch, wobei das Brustpumpenset eine Brusthaube (1) gemäss einem der Ansprüche 1 bis 7 und eine elektrisch betriebene Saugpumpe (4, 4') aufweist.

9. Brustpumpenset nach Anspruch 8, wobei es ferner eine Saugleitung (3) zur Verbindung der Saugpumpe (4) mit der Brusthaube (1) aufweist.

10. Brustpumpenset nach Anspruch 8, wobei die Saugpumpe (4') an der Brusthaube (1) befestigt ist.

11. Brustpumpenset nach einem der Ansprüche 8 bis 10, wobei das Bedienungsmittel (5) über eine Signalleitung (6) mit einer in der Saugpumpe (4, 4') angeordneten Steuerung (42) verbunden ist.

12. Brustpumpenset nach den Ansprüchen 9 und 11, wobei die Signalleitung (6) mit der Saugleitung (3) verbunden ist.

13. Brustpumpenset nach einem der Ansprüche 8 bis 10, wobei das Bedienungsmittel (5) Mittel (50) zur drahtlosen Übermittlung von Signalen an die Saugpumpe (4, 4') aufweist.

14. Brustpumpensets nach einem der Absprüche 8 bis 13, wobei es zwei Brusthauben (1) umfasst und wobei die Saugpumpe (4) zur gleichzeitigen Absaugung von zwei Mutterbrüsten ausgerüstet ist.

15. Brustpumpenset gemäss einem der Ansprüche 8 bis 14, mit einem Verbindungsschlauch, welcher mindestens eine Saugleitung (3) und mindestens eine Signalleitung (6) aufweist.

16. Brustpumpenset nach Anspruch 15, wobei die Saugleitung (3) und die Signalleitung (6) über ihre gesamte Länge miteinander verbunden sind.

## Claims

1. A breastshield of a breastpump set for expressing human breastmilk, wherein the breastshield (1) comprises a breastshield funnel (10) to be placed on a mother's breast, a first coupling part (12) for connection to a milk collection container (2), and a second coupling part (13) for connection to a suction pump (4, 4') and an operating means for operating the suction pump (4, 4'), **characterized in that** the operating means is an operating means (5) for operating the suction pump (4, 4').

2. The breastshield as claimed in claim 1, wherein the second coupling part (13) can be connected to the suction pump (4) via a suction line (3).

3. The breastshield as claimed in claim 1, wherein the second coupling part (13) has means for securing the suction pump (4').

4. The breastshield as claimed in one of claims 1 through 3, wherein the operating means (5) is arranged in the area of the breastshield funnel (10).

5. The breastshield as claimed in claim 4, wherein the operating means (5) is arranged on a breastshield neck (11) adjoining the breastshield (1).

6. The breastshield as claimed in one of claims 4 or 5, wherein, in the position of use, the first coupling part (12) is directed downward and the operating means (5) is arranged on an upwardly directed or downwardly directed or laterally directed area of the breastshield (1).

7. The breastshield as claimed in one of claims 1 through 6, wherein the breastshield (1) has a part which can be plugged on, turned upwards or pulled out and on which the operating means (5) is arranged.

8. A breastpump set for expressing human breastmilk, wherein the breastpump set comprises a breastshield (1) according to one of claims 1 through 7 and an electrically driven suction pump (4, 4').

9. The breastpump set as claimed in claim 8, wherein it further comprises a suction line (3) for connecting the suction pump (4) to the breastshield (1).

10. The breastpump set as claimed in claim 8, wherein the suction pump (4') is secured on the breastshield (1).

11. The breastpump set as claimed in one of claims 8 through 10, wherein the operating means (5) is connected via a signal line (6) to a control system (42) arranged in the suction pump (4, 4').

12. The breastpump set as claimed in claims 9 and 11, wherein the signal line (6) is connected to the suction line (3).

13. The breastpump set as claimed in one of claims 8 through 10, wherein the operating means (5) comprises means (50) for wireless transmission of signals to the suction pump (4, 4').

14. The breastpump set as claimed in one of claims 8 through 13, wherein it comprises two breastshields (1), and wherein the suction pump (4) is equipped for simultaneous suctioning of two breasts.

15. The breastpump set according to one of claims 8 through 14 with a connecting tube which has at least one suction line (3) and at least one signal line (6).

16. The breastpump set according to claim 15, wherein the suction line (3) and the signal line (6) are connected to one another along their entire length.

## Revendications

1. Téterelle d'un ensemble tire-lait pour aspirer du lait maternel humain, dans laquelle la téterelle (1) présente un embout de téterelle (10) à appliquer à un sein maternel, une première pièce de couplage (12) pour le raccordement à un récipient de collecte de lait (2) et une deuxième pièce de couplage (13) pour le raccordement à une pompe aspirante (4, 4') ainsi qu'un moyen de commande, **caractérisée en ce que** le moyen de commande est un moyen de commande (5) pour la commande de la pompe aspirante (4, 4').

2. Téterelle selon la revendication 1, dans laquelle la deuxième pièce de couplage (13) peut être raccordée à la pompe aspirante (4) par une conduite d'aspiration (3).

3. Téterelle selon la revendication 1, dans laquelle la deuxième pièce de couplage (13) présente des moyens pour la fixation de la pompe aspirante (4').

4. Téterelle selon l'une quelconque des revendications 1 à 3, dans laquelle le moyen de commande (5) est disposé dans la région de l'embout de téterelle (10).

5. Téterelle selon la revendication 4, dans laquelle le moyen de commande (5) est disposé sur un col de téterelle (11) adjacent à la téterelle (1).

6. Téterelle selon une des revendications 4 ou 5, dans laquelle en état d'utilisation la première pièce de couplage (12) est dirigée vers le bas et le moyen de commande (5) est disposé sur une région de la téterelle (1) dirigée vers le haut ou dirigée vers le bas ou dirigée latéralement.

7. Téterelle selon l'une quelconque des revendications 1 à 6, dans laquelle la téterelle (1) présente une partie relevable, repliable ou extensible, sur laquelle le moyen de commande (5) est disposé.

8. Ensemble tire-lait pour aspirer du lait maternel humain, dans lequel l'ensemble tire-lait présente une téterelle (1) selon l'une quelconque des revendications 1 à 7 et une pompe aspirante à commande électrique (4, 4').

9. Ensemble tire-lait selon la revendication 8, dans lequel il présente en outre une conduite d'aspiration (3) pour le raccordement de la pompe aspirante (4) à la téterelle (1).

10. Ensemble tire-lait selon la revendication 8, dans lequel la pompe aspirante (4') est fixée à la téterelle (1).

11. Ensemble tire-lait selon l'une quelconque des revendications 8 à 10, dans lequel le moyen de commande (5) est relié à une commande (42) disposée dans la pompe aspirante (4, 4') au moyen d'une ligne de signalisation (6).

12. Ensemble tire-lait selon les revendications 9 et 11, dans lequel la ligne de signalisation (6) est assemblée à la conduite d'aspiration (3).

13. Ensemble tire-lait selon l'une quelconque des revendications 8 à 10, dans lequel le moyen de commande (5) présente des moyens (50) pour la transmission sans fil de signaux à la pompe aspirante (4, 4').

14. Ensemble tire-lait selon l'une quelconque des revendications 8 à 13, dans lequel il comprend deux téterelles (1) et dans lequel la pompe aspirante (4) est équipée en vue de l'aspiration simultanée de deux seins maternels.

15. Ensemble tire-lait selon l'une quelconque des revendications 8 à 14, avec un tuyau flexible de raccordement, qui présente au moins une conduite d'aspiration (3) et au moins une ligne de signalisation (6).

16. Ensemble tire-lait selon la revendication 15, dans lequel la conduite d'aspiration (3) et la ligne de signalisation (6) sont assemblées l'une à l'autre sur toute leur longueur.
